# EUROPEAN PATENT SPECIFICATION

(11) **EP 2 777 480 B1**
(45) Date of publication and mention of the grant of the patent: **02.05.2018**
(21) Application number: 14158740.2
(22) Date of filing: 11.03.2014
(51) Int. Cl.: A61B 1/005

(54) **Flexible shaft with multiple flexible portions**
Biegsame Welle mit mehreren flexiblen Teilen
Arbre flexible avec de multiples parties flexibles

(30) Priority: 12.03.2013 US 201361777261 P; 06.01.2014 US 201414147672
(43) Date of publication of application: 17.09.2014
(73) Proprietor: Covidien LP, Mansfield, MA 02048 (US)
(72) Inventor: Ma, Yong, Cheshire, CT Connecticut 06410 (US)
(74) Representative: Soames, Candida Jane

(56) References cited:
- EP-A1- 2 583 616
- WO-A2-2005/018428
- WO-A2-2013/039999
- US-A1- 2006 178 556
- US-A1- 2009 124 857
- US-A1- 2013 102 846
- US-A1- 2013 190 562

## Description

### BACKGROUND

### Technical Field

The present disclosure relates to surgical devices and, more particularly, to a surgical device including a flexible shaft incorporating multiple flexible portions.

### Background of Related Art

Many surgeons use endoscopic or laparoscopic instruments for remotely accessing organs through smaller, puncture-like incisions or natural orifices. Minimally invasive surgical procedures have been developed during which surgical instruments are passed through small openings in body tissue to access internal surgical sites. Typically, during these procedures, after an incision has been formed in the body tissue, a cannula defining a lumen is inserted through the incision and positioned in relation to the surgical site.

During a laparoscopic procedure, for example, a body cavity is inflated with a nontoxic insufflation gas to create a working area inside a patient for surgical instruments and to allow a trocar to penetrate a body cavity without penetrating an organ within the body cavity. Generally, a plurality of different instruments pass through the cannula to aid the surgeon in performing the surgical procedure.

During a typical minimally invasive procedure, surgical objects, such as surgical access devices (e.g., trocar and cannula assemblies), or endoscopes, are inserted into the patient's body through the incision in tissue. In general, prior to the introduction of the surgical object into the patient's body, insufflation gas are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

While many types of varieties of cannulas and instruments are known, there exists a continuing need for instruments that may be easily maneuverable around other instruments to create a better field of vision for the surgeon.

WO2005/018428A describes an articulated surgical device having the features of the preamble to claim 1.

US2009/124857A describes a flexible steerable medical device comprising a plurality of interconnected segments configured to pivot relative to each other, a flexible tube disposed within the cavities of the plurality of segments, the flexible tube including at least one rigidizing chamber having fusible material disposed therein.

US2006/178556A describes an articulating minimally invasive surgical endoscope with a flexible wrist having at least one degree of freedom.

US2013/190562A, which is equivalent to WO2013/112887A that forms part of the state of the art by virtue of Art. 54(3) EPC, describes a medical device comprising a flexible elongate member including a longitudinal axis and a tool connected to the elongate member near a pivot, wherein the tool may be configured to pivot towards both sides of the longitudinal axis relative to the elongate member at the pivot.

### SUMMARY

In accordance with the present disclosure, a surgical device is provided comprising: a control unit having first, second, third and fourth steering members; and a flexible elongated guide shaft having a proximal end and a distal end, the distal end of the shaft including a camera and the proximal end of the shaft being in operable communication with the control unit; wherein the shaft is partitioned into three segments, the first and second segments operably mated at a first bendable mating point located midway through the shaft, the second and third segments operably mated at a second bendable mating point located at the distal end of the shaft adjacent to the camera; wherein the steering members are positioned within the shaft, the first and second steering members extend through the shaft from the control unit to the first bendable mating point, and the third and fourth steering members extend through the shaft from the control unit to the second bendable mating point, characterized in that: the first, second, third and fourth steering members are separated from each other by 90 degrees; and a fifth steering member is provided for enabling rotational movement of the camera.

Suitably, the camera is remotely controlled. Suitably, the distal end of the shaft includes a plurality of cameras, wherein at least one of the cameras is independently operable from the others.

Suitably, the control unit includes at least one feedback mechanism for providing at least one of substantially continuous and substantially real-time feedback to the control unit.

Suitably, the flexible elongated guide shaft includes at least one illumination source for illuminating a surgical site.

### BRIEF DESCRIPTION OF THE DRAWINGS

Various embodiments of the presently disclosed surgical device with camera assembly, together with reference embodiments outside the scope of the claims, are described hereinbelow with references to the drawings, wherein:
FIG. 1 is a perspective view of a surgical device having its distal end inserted through a cannula assembly and into tissue, in accordance with a reference embodiment of the present disclosure;
FIG. 2 is a perspective view of the surgical device of FIG. 1 having its distal end inserted through the cannula assembly and into tissue, as well as additional devices inserted through the cannula assembly;
FIG. 3A is a perspective view of the surgical device in a first, inactivated position;
FIG. 3B is a perspective view of the surgical device in a second, activated position;
FIG. 4A is a side perspective view of the surgical device illustrating a reference embodiment having a two-member configuration;
FIG. 4B is a cross-sectional view of FIG. 4A at a midpoint of the shaft of the surgical device;
FIG. 5A is a side perspective view of a further reference embodiment of the surgical device illustrating a four-member configuration;
FIG. 5B is a cross-sectional view of FIG. 5A at a midpoint of the shaft of the surgical device;
FIG. 6A is a perspective view of the surgical device reference embodiment having the two-member configuration, where the surgical device is activated in a first, upward direction;
FIG. 6B is a perspective view of the surgical device reference embodiment having the two-member configuration, where the surgical device is activated in a second, downward direction;
FIG. 7 is a perspective view of the surgical device reference embodiment having the four-member configuration, where the surgical device is activated in at least four directions;
FIG. 8 is a perspective view of a further surgical device reference embodiment having at least two flexible portions for enabling bending of at least a portion of the shaft;
FIG. 9A is a perspective view of an embodiment of the surgical device according to the invention having at least two flexible portions for enabling bending and rotating of at least a portion of the shaft;
FIG. 9B is a cross-sectional view of FIG. 9A at one flexible portion of the shaft, in accordance with the present disclosure; and
FIGS. 10A and 10B are perspective views of a surgical device having a plurality of shafts in non-activated and activated positions, in accordance with an embodiment of the present disclosure.

Further scope of applicability of the present disclosure will become apparent from the detailed description given hereinafter. However, it should be understood that the detailed description and specific examples, while indicating embodiments of the present disclosure, are given by way of illustration only, since various changes and modifications within the scope of the accompanying claims will become apparent to those skilled in the art from this detailed description.

### DETAILED DESCRIPTION OF THE EMBODIMENTS

The invention is as disclosed in the appended set of claims. Certain exemplary embodiments and reference embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices specifically described herein and illustrated in the accompanying drawings are non-limiting exemplary embodiments and that the scope of the present disclosure is defined solely by the claims. The features illustrated or described in connection with one exemplary embodiment may be combined with the features of other embodiments. Such modifications and variations are intended to be included within the scope of the present disclosure.

The principles of the present disclosure are applicable to a variety of surgical access devices adapted for permitting percutaneous access to a target site. These access devices include, but are not limited to, trocars and/or cannulas, catheters, hand access devices, scopes, etc. The present disclosure is contemplated for use in various surgical procedures including, e.g., laparoscopic, arthroscopic, thoracic, etc. The present disclosure contemplates the introduction into a body cavity of all types of surgical instruments including clip appliers, graspers, dissectors, retractors, staplers, laser fibers, photographic devices, endoscopes and laparoscopes, tubes, and the like. All such objects, and other similar objects, may be referred to herein as "instrument(s)."

In the figures and in the description that follows, in which like reference numerals identify similar or identical elements, the term "proximal" will refer to the end of the apparatus which is closer to the operator during use, while the term "distal" will refer to the end which is further from the operator.

In the example aspects of the present disclosure, methods and devices are provided for controlling movement of a working end of a surgical device, and in particular for performing various surgical procedures using an instrument having an end effector that is articulated relative to an elongated guide shaft of the device. The end effector also rotates relative to the elongated shaft of the device. The shaft may rotate relative to a handle of the device. Articulation and rotation of the end effector allows the end effector to be positioned at various locations during a surgical procedure, thereby providing the user with precise control over the end effector. The end effector includes a camera.

In an embodiment of the present disclosure, a medical instrument as defined in claim 1 comprising a flexible shaft slidably housed in a flexible sheath is provided, the instrument including a camera carried on a distal end portion of the instrument. The insertable portion of the sheath may comprise a flexible polymer tube. Flexibility of the sheath may be obtained in any number of ways. For example, the sheath may be made of a flexible material, and/or the sheath may be comprised of a number of segments, and/or the sheath may have portions of material removed from it, for example as grooves, to increase flexibility. The instrument may include a functional element disposed on the distal end of the shaft. In the instrument, at least one activation mechanism may be carried along a length of the instrument and may be operatively coupled to the instrument so as to allow a user to control the deflection of at least a distal portion of the instrument. In the instrument, an activation mechanism may be slidably disposed in a channel along at least a portion of the length of the sheath. The instrument may include at least one pullable member, such as a cable, that has a distal portion operatively coupled to a distal end portion of the instrument and a proximal end portion operatively coupled to a tension mechanism at the proximal end of the instrument, the tension mechanism being controllable by a user to cause at least a distal end of the instrument to deflect.

Reference will now be made in detail to embodiments and reference embodiments of the present disclosure. While certain embodiments of the present disclosure will be described, it will be understood that it is not intended to limit the embodiments of the present disclosure to those described embodiments. To the contrary, reference to embodiments of the present disclosure is intended to cover alternatives, modifications, and equivalents as may be included within the scope of the appended claims.

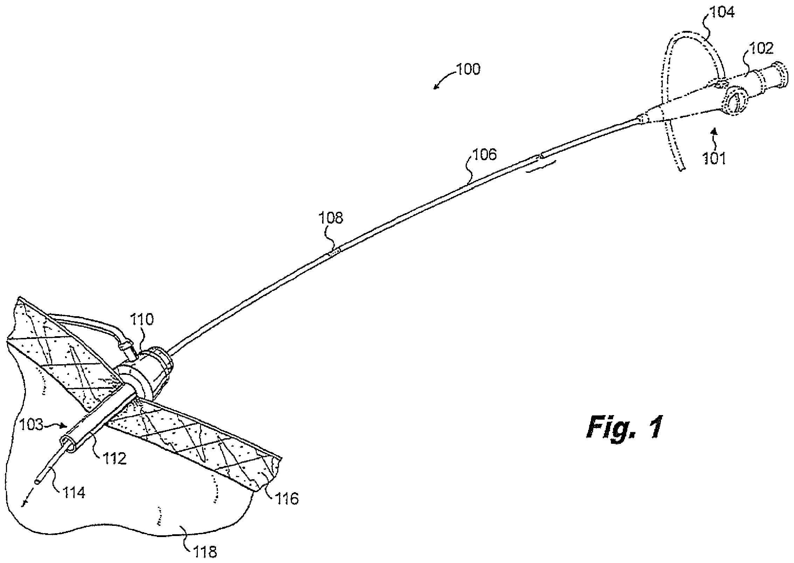

Referring to FIGS. 1-7, reference surgical device system 100 generally includes a shaft 106 having a proximal end 101 and a distal end 103. The proximal end 101 of the shaft 106 includes a handle assembly 102 having an insufflation tube 104. The shaft 106 includes at least one flexible member 108. Flexibility of flexible member 108 may be obtained in any number of ways. For example, member 108 may be made of a flexible material, and/or may be comprised of a number of segments, and/or may have portions of material removed from it, for example as grooves, to increase flexibility. The flexible member 108 may be positioned substantially midway through the shaft 106. The flexible member 108 may be positioned substantially closer to the distal end than the proximal end of the device, or vice versa. In an embodiment, the entire shaft 106 is substantially a flexible member 108. The distal end 103 of the shaft 106 is insertable through a cannula housing 110 having an access member 112. Tip 114 of the shaft 106 extends through the cannula housing 110 and through the access member 112. The access member 112 penetrates tissue 116 of a surgical worksite 118.

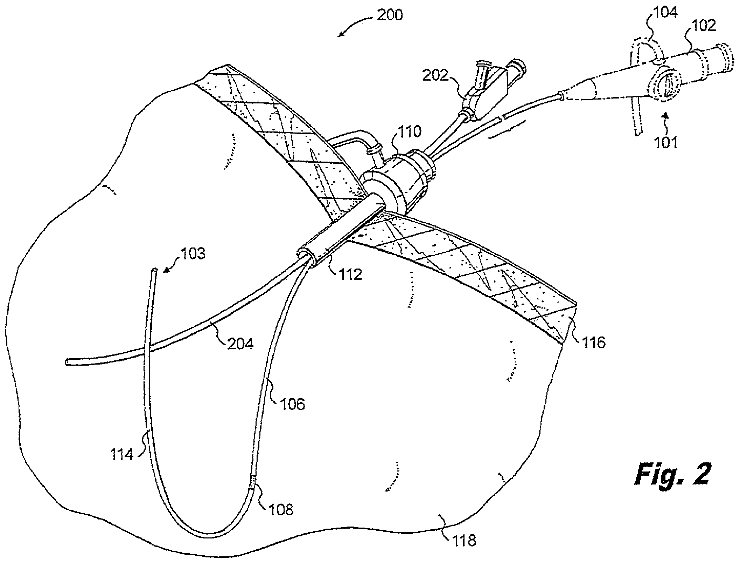

FIG. 1 illustrates the surgical device system 100 having its distal tip 114 inserted through the cannula housing 110 and access member 112, and into tissue 116 of a worksite 118. FIG. 2 illustrates the surgical device system 200 having its distal tip 114 inserted through the cannula housing 110 and access member 112 and into tissue 116, as well as an additional device 202 inserted through the cannula housing 110 and the access member 112, so that the distal tip 204 of the additional device 202 is inserted into the worksite 118. It is contemplated that a plurality of surgical devices may be inserted through the cannula housing 110 and access member 112 and into tissue 116. Such surgical devices may include at least laparoscopes, endoscopes, graspers, scissors, dissectors, retractors, forceps, staplers, clip appliers, light guides, trocars, video systems, surgical robots, and any other type of minimally invasive surgical instrument.

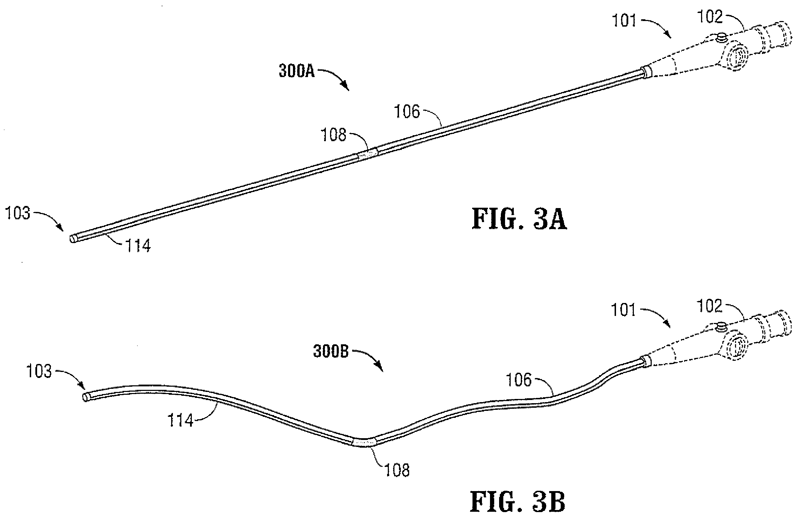

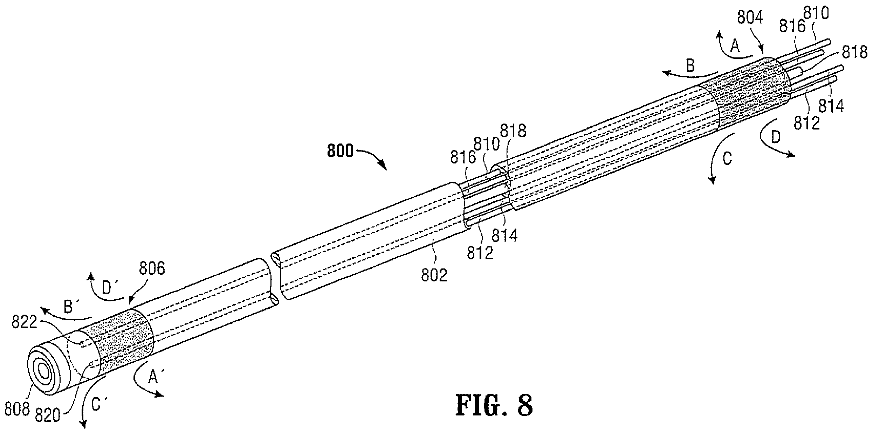

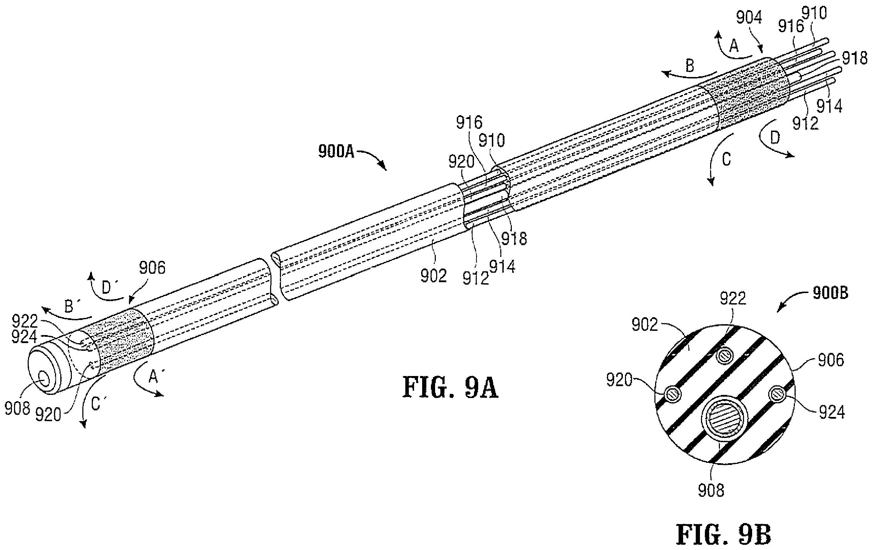

FIG. 3A illustrates the reference surgical device systems 100, 200 of FIGS. 1 and 2 in a first, inactivated position 300A, whereas FIG. 3B illustrates the reference surgical device systems 100, 200 of FIGS. 1 and 2 in a second, activated position 300B. In the first position 300A, the shaft 106 is configured to be in a straight configuration, whereas in the second position 300B, the shaft 106 is configured to be in a bent or curved configuration. The bend occurs at the flexible member 108. The flexible member 108 may be positioned substantially midway through the shaft 106. However, one skilled in the art may contemplate positioning the flexible member 108 on any substantially distal, proximal or midway point across the length of the shaft 106. One skilled in the art may contemplate using a plurality of flexible members across the length of the shaft 106, as described below with reference to FIGS. 8 and 9A. It is also contemplated that the device may include a plurality of flexible members 108, each having a different degree of flexibility and permitting a different degree of bending of the shaft 106. For example, one flexible member positioned closer to the proximal end of the device may be more flexible than a second flexible member positioned closer to the distal end of the device, thus permitting the shaft to bend to a greater degree closer to the proximal end of the device. The reverse is also contemplated.

Additionally, the portions or segments to the left and right of the flexible member 108 are flexible or bendable. The segments 106, 114 may bend in relation to the bend of the flexible member 108. However, the segments 106, 114 may bend independently of the bend of the flexible member 108. One skilled in the art may contemplate a plurality of different bends across the length of each segment 106, 114. One skilled in the art may also contemplate a plurality of different cabling mechanisms for activating the multiple bends across the length of each segment 106, 114.

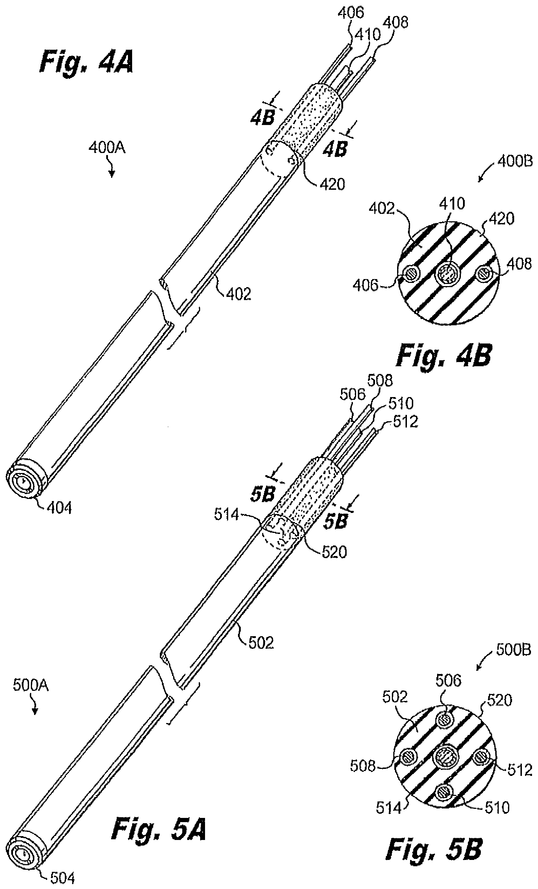

Referring to FIG. 4A, a side perspective view 400A of a portion of the reference surgical device systems 100, 200 of FIGS. 1 and 2 illustrating a two-member configuration is presented.

FIG. 4A depicts a shaft portion 402 adapted to include a camera 404 at its distal end. The proximal end of the shaft portion 402 includes a flexible member 420. The flexible member 420 is actuated by an activation mechanism 410. The activation mechanism 410 includes a first steering member, cable 406 and a second steering member, cable 408. The first member 406 may be substantially diametrically opposed with respect to the second member 408. The first member 406 and the second member 408 commence from the handle assembly 102 (see FIGS. 1 and 2) and terminate substantially at the midpoint of the shaft 402 incorporating the flexible member 420. The first and second members 406 and 408 may operatively cooperate with the activation mechanism 410 to distally move, angularly manipulate, rotate or deflect the distal shaft portion 402 having the camera 404. It is envisioned that the steering member may be a cable, or a bar, or any other well-known device capable of communicating motion.

The activation mechanism 410 may include a pneumatic actuation system (not shown). In an embodiment, a pneumatic actuation system is combined with an electrical control system (not shown) to pneumatically actuate the instruments 100, 200 of FIGS. 1 and 2, and electrically control the flow rate in the pneumatic system. An actuator may be employed to electrically control the pressurization of the pneumatic system. Reference may be made to commonly owned U.S. Patent No. 7,481,347 to Roy filed on March 23, 2005 and commonly owned U.S. Patent No. 8,181,837 to Roy filed on January 5, 2009 , both entitled "Pneumatic Powered Surgical Stapling Device," for a detailed discussion of the construction and operation of exemplary pneumatic actuation systems.

The control system may receive pressurized gas or fluid from a source and produce an electrical output to actuate an element of the instrument employing at least one electrical component or element. The control system may be employed to control elements of a surgical cutting and fastening device and/or a camera. The cutting and fastening elements and/or camera may be pneumatically operable by a controller. Thus, the actuation of a pneumatic cylinder may be controlled with a pressurized gas and a controller. The controller controls the rate at which the pressurized gas is released within the pneumatic system. The controller may be employed to control one or more flow control elements such as solenoids, piezoelectric actuators, or electric motors. These flow control elements may be employed to open and close valves and other closure mechanisms to control the rate of discharge of the pressurized gas into the actuation cylinder. Additional flow control elements may be employed to release the pressurized gas at a variable rate. The embodiments are not limited in this context.

As used herein, the term, "pressurized gas" refers to any gas suitable for use in pneumatically powered systems employed in a sterile environment. Non-limiting examples of such mediums include compressed air, carbon dioxide (CO₂), Nitrogen, Oxygen, Argon, Helium, Sodium Hydride, Propane, Isobutane, Butane Chlorofluorocarbons, Dimethyl ether. It is also envisioned that other fluids or liquids may be utilized.

In an embodiment, a pneumatically powered instrument comprises electrical feedback capabilities. The pneumatically powered instrument may be integrated with a feedback module. The feedback module may be self-contained and adapted to connect to a plurality of contacts disposed throughout the instrument, an electrical circuit element, and a plurality of indicators. The feedback system, indicators, sensors, and controls may be electrically powered and controlled from a remote location. The embodiments are not limited in this context.

The handle assembly 102 (see FIGS. 1 and 2) may supply a support for controls, such as the activation mechanism 410. Activation mechanism 410 provides for a method of controlling the angular deflection of the distal end of the shaft 402 to manipulate the direction of the camera 404. Activation mechanism 410 may include one or more levers, slides, triggers, or similar controls, each attached to at least one deflection member, for example a pull wire, cable or other means for communicating motion from first and second members 406, 408 to the distal end of shaft 402. Extending or retracting first and second members 406, 408 pushes or pulls activation mechanism 410, which slidably extends lengthwise through a proximal end of shaft 402 to one or more points of attachment.

It is also envisioned that the device may include an electromechanical system for controlling the angular deflection of the distal end of the shaft 402 to manipulate the direction of the camera 404. For example, an electronic signal may be sent from the handle assembly 102, down the shaft, for example via wires, to initiate motion in camera 404.

FIG. 4B is a cross-sectional view 400B of the shaft 402 at the flexible member 420. The activation mechanism 410 is substantially centrally disposed within the shaft 402. The first and second members 406, 408 may be located within the shaft and may be substantially diametrically opposed with respect to each other. Of course, one skilled in the art may contemplate a plurality of different positional configurations for the members 406, 408 and the activation mechanism 410. It is envisioned that there may be any number of members and that the members may be operable independent of each other or simultaneously.

Referring to FIG. 5A, a side perspective view 500A of a portion of the surgical device systems 100, 200 illustrating a four-member configuration is presented. The components of FIG. 5A operate similarly to the components of FIG. 4A. However, in contrast to FIG. 4A, FIG. 5A illustrates a four-member configuration.

For example, FIG. 5A depicts a shaft portion 502 adapted to include a camera 504 at its distal end. The proximal end of the shaft portion 502 includes a flexible member 520. The flexible member 520 is actuated by an activation mechanism 514. The activation mechanism 514 includes a first steering member, cable 506, a second steering member, cable 508, a third steering member, cable 510, and a fourth steering member, cable 512. The first member 506 may be substantially diametrically opposed with respect to the second member 508 and the third member 510 may be substantially diametrically opposed to fourth member 512. The first member 506, the second member 508, the third member 510, and the fourth member 512 commence from the handle assembly 102 (see FIGS. 1 and 2) and terminate at the midpoint of the shaft 502 incorporating the flexible member 520. The first, second, third, and fourth members 506, 508, 510, and 512 operatively cooperate with the activation mechanism 514 to angularly manipulate the distal shaft portion 502 having the camera 504. The activation mechanism 514 may be a pneumatic actuation system, as described above with reference to FIG. 4A.

FIG. 5B is a cross-sectional view 500B of the shaft 502 at the flexible member 520. The activation mechanism 514 is centrally disposed within the shaft 502. The first and second members 506, 508 are substantially diametrically opposed with respect to each other, as are the third and fourth members 510, 512. Of course, one skilled in the art may contemplate a plurality of different positional configurations for the members 506, 508, 510, 512 and the activation mechanism 514.

Therefore, at the distal end, an electronic imaging system adapted to acquire an image in a restricted space, such as an internal body cavity or interstitial space is provided. This imaging system, which may be essentially a small video camera 404, 504, is based on a pixilated image sensor such as a Charge Coupled Device (CCD), a Complementary Metal Oxide Semiconductor (CMOS) image sensor, or on a similar analog or digital sensor disposed at or near the distal end of the shaft or sheath constructions. The imaging system typically further comprises one or more optical elements for transmitting an image to the active surface of the image sensor. Such elements may include a protective optical window, objective optics to focus the image on the sensor, and one or more prisms or mirrors to redirect the optical axis by a predetermined angle, alone or in any combination. The imaging system typically further comprises supporting electronics and conductors, for example, to interpret control signals and to transmit image signals to onboard or external equipment.

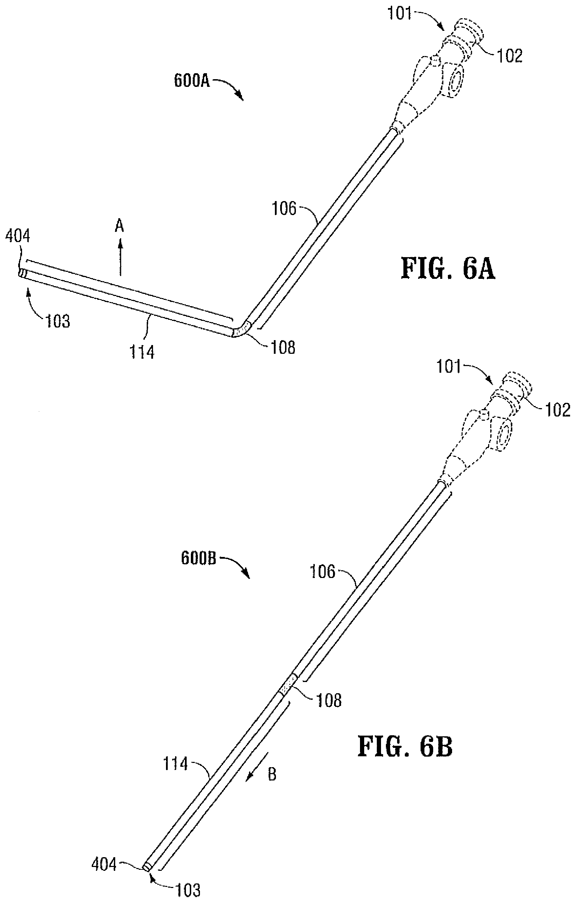

Referring to FIGS. 6A and 6B, a perspective view 600A of the surgical device having the two-member configuration, where the surgical device is activated in a first, upward direction and a perspective view 600B of the surgical device having the two-member configuration, where the surgical device is activated in a second, downward direction, are presented, respectively.

For example, when the first member 406 (see FIG. 4A) is activated by the activation mechanism 410, the distal end 114 of the shaft 106 may bend upward, as shown by arrow "A," in FIG. 6A . When the second member 408 (see FIG. 4A) is activated by the activation mechanism 410, the distal end 114 of the shaft 106 may bend downward, as shown by arrow "B," in FIG. 6B . As a result, the direction of the camera 404 (see FIG. 4A) may be angularly manipulated by the activation mechanism 410 and the push/pull of first and second members 406, 408.

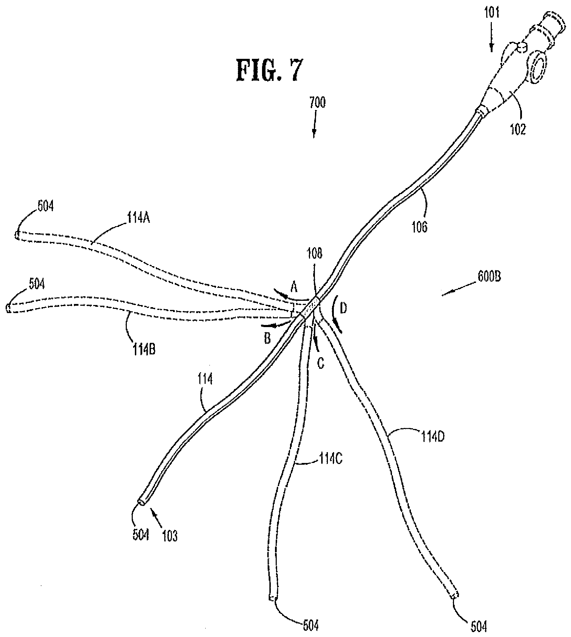

Referring to FIG. 7, a perspective view 700 of the reference surgical device having the four-member configuration, where the surgical device is activated up to four directions, or more, is presented.

For example, when the first member 506 (see FIG. 5A) is activated by the activation mechanism 514, the distal end 114 of the shaft 106 may bend upward, as shown by arrow "A" in the ghost view 114A. When the second member 508 (see FIG. 5A) is activated by the activation mechanism 514, the distal end 114 of the shaft 106 may bend downward, as shown by arrow "C" in the ghost view 114C. When the third member 510 (see FIG. 5A) is activated by the activation mechanism 514, the distal end 114 of the shaft 106 may bend toward the left, as shown by arrow "B" in the ghost view 114B. When the fourth member 512 (see FIG. 5A) is activated by the activation mechanism 514, the distal end 114 of the shaft 106 may bend toward the right, as shown by arrow "D" in the ghost view 114D. As a result, the direction of the camera 504 (see FIG. 5A) may be angularly manipulated by the activation mechanism 514 and the first, second, third, and fourth members 506, 508, 510, and 512.

Additionally, the portions or segments to left and right of the flexible member 108 are flexible or bendable. The segments 106, 114A, B, C, D may bend in relation to the bend of the flexible member 108. However, the segments 106, 114A, B, C, D may bend independently of the bend of the flexible member 108. One skilled in the art may contemplate a plurality of different bends across the length of each segment 106, 114A, B, C, D. One skilled in the art may also contemplate a plurality of different cabling mechanisms for activating the multiple bends across the length of each segment 106, 114A, B, C, D. The multiple bends may bend or flex independently of one another or simultaneously.

Referring to FIG. 8, a perspective view of the reference surgical device having at least two flexible portions for enabling bending of the shaft, in accordance with an embodiment of the present disclosure is presented.

The distal end 800 of the surgical device systems 100, 200 of FIGS. 1 and 2 is illustrated, where the shaft 802 is positioned between a first flexible member 804 and a second flexible member 806. The first flexible member 804 is positioned approximately at a midpoint on the shaft 802, whereas the second flexible member 806 is positioned on the distal end of the shaft 802, adjacent to or in the vicinity of the camera 808.

In FIG. 8, two members terminate at the first flexible member 804 and two members terminate at the second flexible member 806. For example, a first member 810 and a second member 812 may terminate at the first flexible member 804, whereas a third member 814 and a fourth member 816 may terminate at the second flexible member 806, The first member 810 allows the shaft 802 to maneuver in an upward direction, "A," and/or a downward direction "C," whereas the second member 812 allows the shaft 802 to maneuver in a left direction, "B," and/or a right direction, "D," at a substantially midway point of the shaft 802. The third member 814 allows the shaft 802 to maneuver at a distal end in a left direction, "B"' and the fourth member 816 allows the shaft 802 to maneuver at a distal end in a right direction, "A'." Manipulation of multiple members may permit the shaft to maneuver in any combination of these directions, for example an upward and left direction.

Thus, the first and second members 810, 812 maneuver the shaft 802 at the first flexible member 804 and the third and fourth members maneuver the shaft 802 at the second flexible member 806. The first flexible member 804 may be controlled at a midpoint of the shaft 802, whereas the second flexible shaft may be controlled at a distal end of the shaft 802 in order to control the direction of camera 808. As seen in FIG. 8, the distal end 820 of the third member 814 and the distal end 822 of the fourth member 816 terminate at the second flexible member 806 adjacent the camera 808. The first, second, third, and fourth members 810, 812, 814, 816 may be controlled by an activation mechanism 818. The activation mechanism 818 may extend at least a portion of the shaft 802. Exemplary activation mechanisms have been described above with reference to FIGS. 4A and 5A .

FIG. 9A is similar to FIG. 8A. However, in contrast to FIG. 8A, FIG. 9A includes an additional member extending to the distal end of the shaft for enabling rotational movement of the camera.

The distal end 900A of the surgical device systems 100, 200 of FIGS. 1 and 2 is illustrated, where the shaft 902 is positioned between a first flexible member 904 and a second flexible member 906. The first flexible member 904 is positioned approximately at a midpoint on the shaft 902, whereas the second flexible member 906 is positioned on the distal end of the shaft 902, adjacent or in the vicinity of the camera 908.

In FIG. 9A , two members terminate at the first flexible member 904 and two members terminate at the second flexible member 906. For example, a first member 910 and a second member 912 may terminate at the first flexible member 904, whereas a third member 914 and a fourth member 916 may terminate at the second flexible member 906. The first member 910 allows the shaft 902 to maneuver in an upward direction, "A," and/or a downward direction "C," whereas the second member 912 allows the shaft 902 to maneuver in a left direction, "B," and/or a right direction, "D," at a substantially midway point of the shaft 902. The third member 914 allows the shaft 902 to maneuver at a distal end in a left direction, "B"' and the fourth member 916 allows the shaft 902 to maneuver at a distal end in a right direction, "A'."

Thus, the first and second members 910, 912 maneuver the shaft 902 at the first flexible member 904 and the third and fourth members maneuver the shaft 902 at the second flexible member 906. The first flexible member 904 may be controlled at a midpoint of the shaft 902, whereas the second flexible shaft may be controlled at a distal end of the shaft 902 in order to control the direction of camera 908. As seen in FIG. 9A, the distal end 920 of the third member 914 and the distal end 922 of the fourth member 916 terminate at the second flexible member 906 adjacent the camera 908. Additionally, a fifth member 924 may extend the length of the shaft 902 and terminate at the second flexible member 906. The fifth member 924 may be used to control the rotation of the camera 908.

The first, second, third, fourth, and fifth members 910, 912, 914, 916, 924 may be controlled by an activation mechanism 918. The activation mechanism 918 may extend at least a portion of the shaft 902. Example activation mechanisms have been described above with reference to FIGS. 4A and 5A.

FIG. 9B illustrates a cross sectional view 900B of the shaft 902 at the second flexible member 906. The cross-sectional view 900B illustrates the positioning of the members, as well as the positioning of the camera 908. Of course, one skilled in the art may contemplate a plurality of different positional configurations for the members 910, 912, 914, 916 and the activation mechanism 918 and the camera 908.

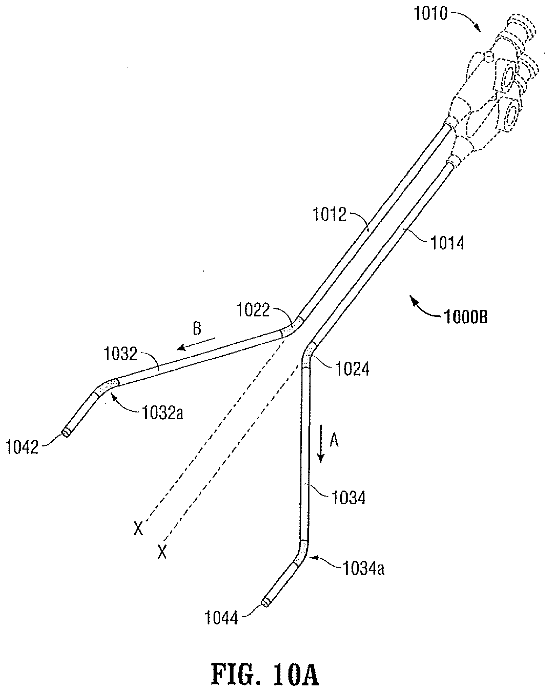

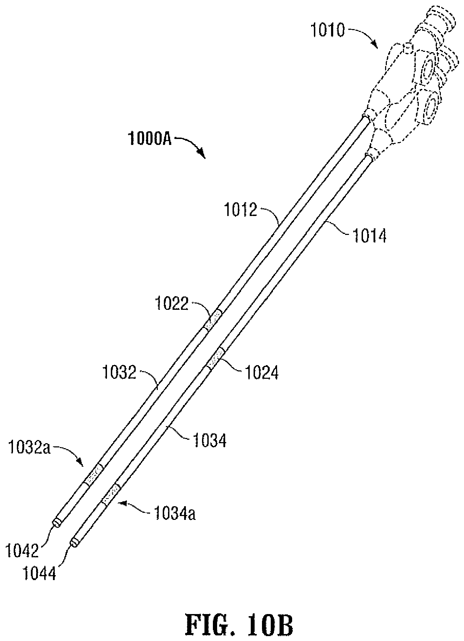

Referring to FIGS. 10A and 10B, in another example embodiment, perspective views 1000A, 1000B of a surgical device having a plurality of shafts in non-activated and activated positions is presented.

In an inactivated position 1000A, the surgical device 1010 includes a first shaft 1012 and a second shaft 1014. The first shaft 1012 includes a flexible member 1022 and a distal end 1032 including a first camera 1042. The second shaft 1014 includes a flexible member 1024 and a distal end 1034 including a first sensor or camera 1044. In an activated position 1000B, the first shaft 1012 may bend at the flexible member 1022 in a first direction "B," whereas the second shaft 1014 may bend at the flexible member 1024 in a second direction "A." The shafts 1012, 1014 may be spaced apart from each other or may be substantially adjacent to each other. In an embodiment, the shafts 1012, 1014 are positioned directly next to each other. One skilled in the art may contemplate positioning a plurality of different shafts in a number of configurations, where at least one shaft includes a camera at its distal end.

In summary, the disclosed embodiments of the present disclosure may help provide an effective surgical device that enhances the maneuvering of cameras within the surgical worksite.

It will be understood that there are to be no limitations as to the dimensions and shape of the shaft and/or members and/or cameras or the materials from which the shaft and/or members and/or cameras are manufactured or the electronics that may be used to run such a camera assembly. It is to be realized that the optimum dimensional relationships for the parts of the present disclosure, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present disclosure.

From the foregoing and with reference to the various figure drawings, those skilled in the art will appreciate that certain modifications may also be made to the present disclosure without departing from the scope of the accompanying claims. Therefore, the above description should not be construed as limiting, but merely as exemplifications of particular embodiments. Those skilled in the art will envision other modifications within the scope of the claims appended hereto.

## Claims

1. A surgical device comprising:
a control unit having first, second, third and fourth steering members (910,912,914,916); and
a flexible elongated guide shaft (902) having a proximal end and a distal end, the distal end of the shaft including a camera (908) and the proximal end of the shaft being in operable communication with the control unit;
wherein the shaft is partitioned into three segments, the first and second segments operably mated at a first bendable mating point (904) located midway through the shaft, the second and third segments operably mated at a second bendable mating point (906) located at the distal end of the shaft adjacent to the camera (908);
wherein the steering members are positioned within the shaft, the first (910) and second (912) steering members extend through the shaft from the control unit to the first bendable mating point (904), and the third (914) and fourth (916) steering members extend through the shaft from the control unit to the second bendable mating point (906),
**characterized in that**:
the first, second, third and fourth steering members are separated from each other by 90 degrees; and
a fifth steering member (924) is provided for enabling rotational movement of the camera (908).

2. The surgical device according to claim 1, wherein the distal end of the shaft includes a plurality of cameras, wherein at least one of the cameras is independently operable from the others.

3. The surgical device according to claim 1, wherein the camera (908) is remotely controlled.

4. The surgical device according to any preceding claim, wherein the control unit includes at least one feedback mechanism for providing at least one of continuous and real-time feedback to the control unit.

5. The surgical device according to any preceding claim, wherein the distal end of the flexible elongated guide shaft (902) includes at least one illumination source for illuminating a surgical site.

## Patentansprüche

1. Chirurgische Vorrichtung umfassend:
eine Steuereinheit mit ersten, zweiten, dritten und vierten Steuerelementen (910, 912, 914, 916); und
einen flexiblen langgestreckten Führungsschaft (902) mit einem proximalen Ende und einem distalen Ende, wobei das distale Ende des Schaftes eine Kamera (908) aufweist und das proximale Ende des Schaftes mit der Steuereinheit in betriebsfähiger Verbindung steht;
wobei der Schaft in drei Segmente unterteilt ist, wobei die ersten und zweiten Segmente an einer ersten biegbaren Fügestelle (904), die mittig durch den Schaft angeordnet ist, betriebsfähig zusammengefügt sind, wobei die zweiten und dritten Segmente an einer zweiten biegbaren Fügestelle (906), die an dem distalen Ende des Schaftes benachbart zu der Kamera (908) angeordnet ist, betriebsfähig zusammengefügt sind;
wobei die Steuerelemente innerhalb des Schaftes positioniert sind, wobei sich die ersten (910) und zweiten (912) Steuerelemente durch den Schaft von der Steuereinheit zu der ersten biegbaren Fügestelle (904) erstrecken und sich die dritten (914) und vierten (916) Steuerelemente durch den Schaft von der Steuereinheit zu der zweiten biegbaren Fügestelle (906) erstrecken,
**dadurch gekennzeichnet, dass**:
die ersten, zweiten, dritten und vierten Steuerelemente um 90 Grad voneinander getrennt sind; und
ein fünftes Steuerelement (924) vorgesehen ist, um eine Rotationsbewegung der Kamera (908) zu ermöglichen.

2. Chirurgische Vorrichtung nach Anspruch 1, wobei das distale Ende des Schaftes eine Vielzahl von Kameras aufweist, wobei zumindest eine der Kameras unabhängig von den anderen betrieben werden kann.

3. Chirurgische Vorrichtung nach Anspruch 1, wobei die Kamera (908) ferngesteuert ist.

4. Chirurgische Vorrichtung nach einem der vorstehenden Ansprüche, wobei die Steuereinheit zumindest einen Feedback-Mechanismus aufweist, um zumindest eines von kontinuierlichem und Echtzeit-Feedback an die Steuereinheit bereitzustellen.

5. Chirurgische Vorrichtung nach einem der vorstehenden Ansprüche, wobei das distale Ende des flexiblen langgestreckten Führungsschaftes (902) zumindest eine Beleuchtungsquelle zum Beleuchten einer Eingriffsstelle aufweist.

## Revendications

1. Dispositif chirurgical comprenant :
une unité de commande ayant un premier, un deuxième, un troisième et un quatrième élément de guidage (910, 912, 914, 916) ; et
un arbre de guidage allongé souple (902) ayant une extrémité proximale et une extrémité distale, l'extrémité distale de l'arbre comprenant une caméra (908) et l'extrémité proximale de l'arbre étant en communication de fonctionnement avec l'unité de commande ;
dans lequel l'arbre est séparé en trois segments, le premier et le deuxième segment étant accouplés en service en un premier point d'accouplement repliable (904) situé à mi-chemin à travers l'arbre, les deuxième et troisième segment étant accouplés en service en un deuxième point d'accouplement repliable (906) situé à l'extrémité distale de l'arbre adjacente à la caméra (908) ;
dans lequel les éléments de direction sont positionnés dans l'arbre, le premier (910) et le deuxième (912) élément de direction s'étendent à travers l'arbre de l'unité de commande au premier point d'accouplement repliable (904) et le troisième (914) et le quatrième (916) élément de direction s'étendent à travers l'arbre de l'unité de commande au deuxième point d'accouplement repliable (906),
**caractérisé en ce que** :
les premier, deuxième, troisième et quatrième éléments de direction sont séparés l'un de l'autre de 90 degrés ; et
un cinquième élément de direction (924) est prévu pour permettre un mouvement rotatif de la caméra (908).

2. Dispositif chirurgical selon la revendication 1, dans lequel l'extrémité distale de l'arbre comprend une pluralité de caméras, dans lequel au moins l'une des caméras peut fonctionner indépendamment des autres.

3. Dispositif chirurgical selon la revendication 1, dans lequel la caméra (908) est commandée à distance.

4. Dispositif chirurgical selon l'une quelconque des revendications précédentes, dans lequel l'unité de commande comprend au moins un mécanisme de rétroaction pour fournir au moins l'une d'une rétroaction continue et d'une rétroaction en temps réel à l'unité de commande.

5. Dispositif chirurgical selon l'une quelconque des revendications précédentes, dans lequel l'extrémité distale de l'arbre de guidage allongé souple (902) comprend au moins une source d'éclairage pour éclairer un site chirurgical.
